# EUROPÄISCHE PATENTSCHRIFT

(11) **EP 1 922 287 B1**
(45) Veröffentlichungstag und Bekanntmachung des Hinweises auf die Patenterteilung: **23.12.2015**
(21) Anmeldenummer: 06792949.7
(22) Anmeldetag: 22.08.2006
(51) Int. Cl.: C01B 17/16, C07C 319/08, C07C 321/04

(54) **VERFAHREN ZUR HERSTELLUNG VON METHYLMERCAPTAN**
PROCESS FOR PREPARING METHYL MERCAPTAN
PROCEDE DE PRODUCTION DE METHYLMERCAPTANE

(30) Priorität: 10.09.2005 DE 102005043151
(43) Veröffentlichungstag der Anmeldung: 21.05.2008
(73) Patentinhaber: Evonik Degussa GmbH, 45128 Essen (DE)
(72) Erfinder: REDLINGSHÖFER, Hubert, 91481 Münchsteinach (DE); KRETZ, Stephan, 63599 Biebergemünd (DE); FINKELDEI, Caspar-Heinrich, 63755 Alzenau (DE); WECKBECKER, Christoph, 63584 Gründau-Lieblos (DE); BÖCK, Wolfgang, 63505 Langenselbold (DE); HUTHMACHER, Klaus, 63571 Gelnhausen (DE)
(86) Internationale Anmeldenummer: PCT/EP2006/065565
(87) Internationale Veröffentlichungsnummer: WO 2007/028708

(56) Entgegenhaltungen:
- WO-A-01/10776
- DE-C1- 19 654 515
- GB-A- 1 193 040
- US-A- 2 863 725

## Beschreibung

Die vorliegende Erfindung betrifft ein Verfahren zur kontinuierlichen Herstellung von Methylmercaptan aus Schwefelwasserstoff und Methanol in direkter Verbindung mit der Herstellung von Schwefelwasserstoff.

Insbesondere Methylmercaptan ist ein industriell wichtiges Zwischenprodukt zum Beispiel für die Synthese von Methionin sowie für die Synthese von Dimethylsulfoxid und Dimethylsulfon. Es wird heute überwiegend aus Methanol und Schwefelwasserstoff durch Reaktion an einem Katalysator aus Aluminiumoxid hergestellt. Die Synthese des Methylmercaptans erfolgt gewöhnlich in der Gasphase bei Temperaturen zwischen 300 und 500°C und bei Drücken zwischen 1 und 50 bar.

Das Produktgasgemisch enthält neben dem gebildeten Methylmercaptan und Wasser die nicht umgesetzten Ausgangsstoffe Methanol und Schwefelwasserstoff und als Nebenprodukte Dimethylsulfid und Dimethylether, sowie in geringen Mengen auch Polysulfide (Dimethyldisulfid). Im Sinne der Reaktion inerte Gase wie Kohlenmonoxid, Kohlendioxid, Stickstoff und Wasserstoff sind auch im Produktgas enthalten. Aus diesem Reaktionsgemisch wird das gebildete Methylmercaptan abgetrennt. Das Eduktgasgemisch enthält vorwiegend Schwefelwasserstoff und Methanol in einem molaren Verhältnis zwischen 1:1 und 10:1.

Aus dem Produktgasgemisch wird das gebildete Methylmercaptan, wie in DE 1768826 erläutert, in mehreren Destillations- und Waschkolonnen bei Temperaturen zwischen 10 und 140°C abgetrennt. Dabei fallen als weitere Produktströme überschüssiger Schwefelwasserstoff, Methanol, Inertgase wie Kohlenmonoxid, Kohlendioxid, Stickstoff und Wasser an. Als Waschflüssigkeit wird bevorzugt Methanol verwendet. Überschüssiger Schwefelwasserstoff wird als sogenanntes Kreisgas in den Reaktor zurückgeführt. Das Kreisgas enthält neben Schwefelwasserstoff noch Methanol, Methylmercaptan, Dimethylsulfid und organische Komponenten, wobei verbrauchter Schwefelwasserstoff und Methanol durch Zuführen von frischen Medien ersetzt werden.

Der Gesamtprozess der Methylmercaptan-Herstellung kann in zwei Abschnitte unterteilt werden. Der erste Abschnitt umfasst die Aufbereitung des Eduktgasgemisches und dessen Umsetzung zu Methylmercaptan. Der zweite Bereich beinhaltet die Trennung des Produktgasgemisches zur Gewinnung von Methylmercaptan und Rückführung der nicht verbrauchten Einsatzstoffe, sowie die Entsorgung von Abwasser und Abgasen.

Für die Wirtschaftlichkeit des Verfahrens werden möglichst niedrige Investitions- und Betriebskosten gefordert. Hier stellt insbesondere der Aufwand für Apparate und Maschinen aber auch der Energieaufwand zur Synthese bzw. Aufbereitung des Eduktgasgemisches einen hohen Kostenfaktor dar. Z.B. werden große elektrische Leistungen für den Betrieb von Verdichtern und von Heiz- und Kühlkreisläufen benötigt. Gemäß der FR 2477538 wird zur Herstellung von Methylmercaptan frisches Schwefelwasserstoffgas in einem Verdichter auf 11 bar verdichtet. Danach werden aus dem Prozess zurückgeführtes Kreisgas, welches Schwefelwasserstoff, Dimethylsulfid, Methanol und geringe Mengen Methylmercaptan enthält, dem verdichteten Schwefelwasserstoff zur Bildung des Eduktgasgemisches zugeführt. Durch einen Vorwärmofen wird die Temperatur der Gasmischung nach der Verdichtung auf 510°C angehoben.

Die WO 01/10776 A1 offenbart ein zweistufiges Verfahren zur Herstellung von Methylmercaptan, bei dem zunächst durch reduktive Verbrennung eines Ammoniumsalzes von Schwefelsäure Schwefelwasserstoff hergestellt wird, der anschließend durch Reaktion mit Methanol in Methylmercaptan umgewandelt wird. Diese Verfahrensweise erfordert allerdings den Betrieb von Verdichtern, was mit einen hohen energie- und kostenintensiven Aufwand verbunden ist. Dies macht jedoch das Verfahren der WO 01/10776 A1 für eine industrielle Anwendung unattraktiv.

Auch in der DE 19654515 wird die Verdichtung der Eduktgase auf Betriebsdruck bevorzugt in zwei Stufen beschrieben, z.B. mit einem zweistufigen Verdichter, wobei das Gasgemisch in der ersten Stufe auf einen Zwischendruck und in der zweiten Stufe auf den Betriebsdruck komprimiert wird. Das Methanol kann direkt in die erste Verdichterstufe eingespritzt werden. Das so erhaltene Eduktgasgemisch wird dann zunächst auf eine Vortemperatur von 150 bis 250°C und dann weiter auf Reaktionstemperatur erwärmt. Mit dieser Temperatur gelangt das Eduktgasgemisch in den Reaktor zur Bildung von Methylmercaptan. Aufgrund der Temperaturbegrenzung bei einer Verdichtung kann die Temperatur nach der zweiten Verdichterstufe auf maximal 140 °C gesteigert werden.

Dies bedeutet, dass die Eingangstemperatur des Schwefelwasserstoffs vor der Verdichtung z.B. bei Umgebungstemperatur liegen muss. Folglich muss der zuvor bei hoher Temperatur hergestellte Schwefelwasserstoff zunächst abgekühlt und nach der Verdichtung zum Erreichen der Reaktionstemperatur für die Bildung von Methylmercaptan erneut aufgeheizt werden. Dieses Abkühlen und wiederholte Aufheizen erfordert zahlreiche Wärmetauscher und hohe Energiekosten. Weiterhin sollte der Schwefelwasserstoff zur Verdichtung keine Verunreinigungen oder gar Feststoffe enthalten, um den Verdichter nicht zu schädigen.

Die Synthese von Schwefelwasserstoff aus den Elementen Wasserstoff und Schwefel erfolgt üblicherweise durch Einleiten von Wasserstoff in flüssigen Schwefel und einem nachgeschalteten Reaktionsraum in der Gasphase. Dabei sind sowohl katalysierte als auch unkatalysierte Verfahren bekannt.

Die industrielle Produktion von Schwefelwasserstoff aus den Elementen verläuft nach Ullmann's Encyclopedia of Industrial Chemistry, Wiley-VCH, 2002 bei Temperaturen von 450 °C und einem Druck von 7 bar.

CSSR 190792 beschreibt eine Verfahrensvariante zur Herstellung von Schwefelwasserstoff, wobei hohe Reaktionstemperaturen durch eine vergleichsweise aufwendige Reihenschaltung von mehreren Reaktoren vermieden werden. Hohe Temperaturen werden dort speziell aufgrund von Korrosionsproblemen vermieden.

In der GB 1193040 ist die unkatalysierte Synthese von Schwefelwasserstoff bei relativ hohen Temperaturen von 400 bis 600 °C und Drücken von 4 bis 15 bar beschrieben. Es wird angegeben, dass die erforderliche Temperatur vom Druck bestimmt wird, bei dem die Synthese ablaufen soll. Bei einem Druck von 9 bar sind demnach etwa 500 °C erforderlich.

Insgesamt gibt es zahlreiche Veröffentlichungen mit verschiedenen Katalysatoren zur Herstellung von Schwefelwasserstoff. So beschreibt US 2214859 den Einsatz mehrerer unterschiedlicher Metalloxide und Metallsulfide mit hohen Umsatzgraden an Wasserstoff. In der US 2863725 wird die Verwendung von Katalysatoren wie Molydänsulfid, Kobaltoxid oder Kobaltmolybdat gebunden auf Trägern wie Bauxit oder Aluminiumoxid beschrieben, um möglichst schwefelfreien Schwefelwasserstoff herzustellen.

Ein wesentlicher Punkt bei der Herstellung von Schwefelwasserstoff aus Schwefel und Wasserstoff ist vor allem die Temperaturführung. Hohe Temperaturen sind notwendig, um einen Gleichgewichtszustand zu erreichen, bei dem sich ein Molverhältnis Wasserstoff:Schwefel in der Gasphase von etwa 1:1 einstellt. Erst dies ermöglicht die Synthese von reinem Schwefelwasserstoff. Mit zunehmendem Druck ist die Temperatur entsprechend der Dampfdruckkurve von Schwefel stark zu erhöhen, um das angestrebte Molverhältnis von 1:1 in der Gasphase zu erreichen. Dabei sind schon geringe Unterschiede im Druck von z. B. 1 bar und weniger von großer Bedeutung.

Aufgabe der Erfindung ist es, ein neues Verfahren zur Herstellung von Methylmercaptan bereitzustellen. Gegenstand der Erfindung ist ein Verfahren zur Herstellung von Methylmercaptan, das dadurch gekennzeichnet ist, dass man die Synthesen von Schwefelwasserstoff und Methylmercaptan miteinander koppelt, indem man das unter Druck aus dem Reaktor für die Schwefelwasserstoffsynthese austretende Reaktionsgemisch mit Methanol mischt und unter Druck in den Reaktor zur Methylmercaptansynthese einleitet, wobei man zwischen den für die beiden Synthesen verwendeten Reaktoren einen Druckunterschied einstellt, der dass Schwefelwasserstoff/Methanol-Gemisch (Eduktgas)in Richtung des Methylmercaptanreaktors strömen lässt.

Dieser Druckunterschied beträgt dabei im allgemeinen weniger als 1 bar, bevorzugt weniger als 0,6 bar und ist immer größer als 0 bar, wobei in dem Reaktor für die Schwefelwasserstoffsynthese der höhere Druck herrscht.

Die erfindungsgemäße Verbindung der Reaktoren für die Schwefelwasserstoff- und Methylmercaptansynthese, bei der das aus dem Schwefelwasserstoffreaktor austretende Reaktionsgemisch einen im Vergleich zum Methylmercaptanreaktor um >0 bis 1 bar höheren Druck aufweist, gestattet es, die notwendige Verdichtung des Schwefelwasserstoffs, wie sie aus dem Stand der Technik bekannt ist, zu vermeiden. In der Eduktgasaufbereitung kann erfindungsgemäß auch auf das Abkühlen auf Umgebungstemperatur und auf das erneute Aufheizen verzichtet werden. Ferner stören auch kleine Mengen an Verunreinigungen und Restmengen an Schwefel eine kontinuierliche Produktion nicht, da der dafür störanfällige Verdichter erfindungsgemäß nicht erforderlich ist. Durch den höheren Druck in der Eduktgasaufbereitung wird auch die Gasdichte in den Apparaten erhöht, was eine kompaktere Bausweise bei konstanter Verweilzeit ermöglicht.

Der Fachmann ist in der Auswahl der zu kombinierenden Verfahrensschritte zur Herstellung von Schwefelwasserstoff frei.

In einer Ausführungsform zur Herstellung von Schwefelwasserstoff wird Wasserstoff bei einem Druck von 8 bis 20 bar in flüssigen Schwefel eingeleitet und in einen nachgeschalteten Reaktionsraum umgesetzt. Die gesamte Anordnung wird bevorzugt bei der gleichen Temperatur betrieben.

Weiterhin läuft die Umsetzung zu Schwefelwasserstoff bevorzugt in Gegenwart eines heterogenen Katalysators ab. Dabei handelt es sich um einen schwefelbeständigen Hydrierkatalysator, der bevorzugt aus einem Träger wie beispielsweise Siliziumoxid, Aluminiumoxid, Zirkoniumoxid oder Titanoxid besteht sowie eines oder mehrere der aktiven Elemente Molybdän, Nickel, Wolfram, Vanadium, Kobalt, Schwefel, Selen, Phosphor, Arsen, Antimon, Wismut, Silizium, Aluminium, Titan und Zirkonium enthält. Der Katalysator kann sowohl in der Flüssigphase als auch in der Gasphase eingesetzt werden. Je nach Reaktionsbedingungen, insbesondere bei hohen Temperaturen kann ein Teil des Schwefelwasserstoffs auch ohne die Einwirkung eines Katalysators gebildet werden.

In einer weiteren Ausführungsform der Erfindung werden mehrere, insbesondere zwei oder drei Reaktoren in Serie geschaltet. Dabei wird der dann nur teilweise umgesetzte Wasserstoff zusammen mit dem gebildeten Schwefelwasserstoff in einem weiteren Reaktor zur weiteren Umsetzung zu Schwefelwasserstoff bevorzugt in flüssigem Schwefel verteilt und direkt im Bereich des flüssigen Schwefels und/oder in einem nachgeschalteten Gasraum weiter zu Schwefelwasserstoff umgesetzt. Bei der Verwendung von zwei in Reihe geschalteten Reaktoren beträgt der Umsatz von Wasserstoff nach dem ersten Reaktor im allgemeinen zwischen 40 und 85 %. Werden drei Reaktoren eingesetzt, so beträgt der Umsatz von Wasserstoff nach dem ersten Reaktor 20 bis 50 % und nach dem zweiten Reaktor im allgemeinen 50 bis 85 %.

Anstelle von reinem Wasserstoff kann auch verunreinigter Wasserstoff durch den flüssigen Schwefel geleitet werden. Die Verunreinigungen können beispielsweise Kohlendioxid, Schwefelwasserstoff, Wasser, Methanol, Methan, Ethan, Propan, oder andere leichtflüchtige Kohlenwasserstoffe sein. Bevorzugt wird Wasserstoff mit einer Reinheit größer als 65 Vol.-% eingesetzt, wovon bevorzugt mehr als 98 % des eingesetzten Wasserstoffs zu Schwefelwasserstoff umgesetzt werden. Die Verunreinigungen im Wasserstoff oder deren Reaktionsprodukte werden bevorzugt vor der Synthese von Methylmercaptan nicht abgetrennt, sondern im Eduktgemisch belassen.

Um die Verluste an Schwefel zu minimieren, wird der überwiegende Teil des nicht zu Schwefelwasserstoff umgesetzten Schwefels aus dem Schwefelwasserstoff vor dessen Umsetzung zu Methylmercaptan abgetrennt und zurückgeführt. Dies erfolgt beispielsweise durch Abscheiden von Schwefel an Wärmetauscherflächen, durch eine Adsorption oder durch eine Absorption. Die Temperatur ist dabei bevorzugt so einzustellen, dass der Schwefel flüssig abgetrennt werden kann. Dazu werden Temperaturen zwischen 120 und 300 °C bevorzugt. Die Abtrennung von Schwefel und/oder schwefelhaltigen Verbindungen erfolgt bei einem Druck, der zwischen den bei der Synthese von Schwefelwasserstoff und Methylmercaptan eingestellten Drücken liegt. Schwefelwasserstoff wird erfindungsgemäß bevorzugt im Druckbereich von >9 bis 20 bar und Methylmercaptan im Druckbereich von 9 bis <20 bar hergestellt, wobei der Druck im Schwefelwasserstoffreaktor immer den höheren Wert einnimmt.

Insgesamt können durch die Erfindung zahlreiche teilweise sehr aufwendige Apparate und Maschinen sowie Energiekosten eingespart werden, was die Kosten der Synthese von Methylmercaptan deutlich senkt, die Wirtschaftlichkeit verbessert sowie die Verfügbarkeit von Produktionsanlagen steigert.

### Beispiel

Wasserstoff wurde kontinuierlich bei einem Druck von 12,2 bar in einen Reaktor, der bis etwa zur Hälfte mit flüssigem Schwefel gefüllt war, über eine Fritte (100 µm) in die Flüssigkeit eingeleitet und mit gasförmigem Schwefel gesättigt. Im Reaktor, der gleichmäßig bei 450 °C beheizt wurde, befand sich eine von der Gasphase durchströmte Schüttung eines handelsüblichen Hydrierkatalysators (Co- und Mo-Oxid gebunden mit Al₂O₃). Die Analyse mittels Gaschromatographie ergab einen Umsatzgrad von Wasserstoff von mehr als 99 %. Das den Reaktor verlassende Gas wurde nicht entspannt und in einem Wärmetauscher auf ca. 170 °C abgekühlt. Dabei abgetrennter flüssiger Schwefel wurde in den Reaktor zurückgeleitet. Der Wärmeinhalt des bei 12,2 bar erzeugte Schwefelwasserstoff wurde zur Verdampfung von Methanol genutzt. Das so Schwefelwasserstoff und Methanol enthaltende Eduktgasgemisch wurde bei 340 °C in den bei unter 12 bar betriebenen Reaktor zur Umsetzung zu Methylmercaptan geleitet. In diesem Reaktor wurde ein Alkali-Wolframat-Katalysator gemäß DE 10338887 eingesetzt. Insgesamt wurde der einleitete Wasserstoff mit einer konstanten Selektivität von ca. 97 % zu Methylmercaptan umgesetzt. Der kontinuierliche Prozess wurde ohne Störungen 500 h betrieben.

## Patentansprüche

1. Verfahren zur Herstellung von Methylmercaptan **dadurch gekennzeichnet, dass** man die Synthesen von Schwefelwasserstoff und Methylmercaptan miteinander koppelt, indem man das unter Druck aus dem Reaktor für die Schwefelwasserstoffsynthese austretende Reaktionsgemisch mit Methanol mischt und unter Druck in den Reaktor zur Methylmercaptansynthese einleitet" wobei man zwischen den für die beiden Synthesen verwendeten Reaktoren einen Druckunterschied einstellt, der das Schwefelwasserstoff/Methanol-Gemisch in Richtung des Methylmercaptanreaktors strömen lässt.

2. Verfahren gemäß Anspruch 1, **dadurch gekennzeichnet, dass** der Druckunterschied in den für die beiden Synthesen eingesetzten Reaktoren >0 bis <1 bar beträgt, wobei in dem Reaktor für die Schwefelwasserstoffsynthese der höhere Druck herrscht.

3. Verfahren gemäss den Ansprüchen 1 oder 2, **dadurch gekennzeichnet, dass** der Druck in den Reaktionsgefäßen bei beiden Verfahren mehr als 8 bar beträgt.

4. Verfahren gemäss den Ansprüchen 1 bis 3, **dadurch gekennzeichnet, dass** sich der Druck in dem Reaktor für die Schwefelwasserstoffsynthese auf >9 bis 20 bar und im Methylmercaptanreaktor auf 9 bis <20 bar beläuft.

5. Verfahren gemss den Ansprüchen 1 bis 4, **dadurch gekennzeichnet, dass** die Synthese von Schwefelwasserstoff bei einer Temperatur zwischen 300 und 500°C erfolgt.

6. Verfahren gemäss den Ansprüchen 1 bis 5, **dadurch gekennzeichnet, dass** die Synthese von Schwefelwasserstoff in Gegenwart eines heterogenen Katalysators erfolgt.

7. Verfahren gemäss Anspruch 6, **dadurch gekennzeichnet, dass** die Synthese von Schwefelwasserstoff in Gegenwart eines heterogenen Hydrierkatalysators erfolgt.

8. Verfahren gemäss Anspruch 6, **dadurch gekennzeichnet, dass** die Synthese von Schwefelwasserstoff in Gegenwart eines heterogenen Trägerkatalysators erfolgt, der eines oder mehrere der aktiven Elemente, ausgewählt aus der Gruppe Molybdän, Nickel, Wolfram, Vanadium, Kobalt, Schwefel, Selen, Phosphor, Arsen, Antimon, Wismut, Silizium, Aluminium, Titan und Zirkonium enthält.

9. Verfahren gemäss den Ansprüchen 1 bis 8, **dadurch gekennzeichnet, dass** der Schwefelwasserstoff in zwei oder mehreren in Reihe geschalteten Reaktoren hergestellt wird.

10. Verfahren gemäss den Ansprüchen 1 bis 9, **dadurch gekennzeichnet, dass** der zur Herstellung von Schwefelwasserstoff eingesetzte Wasserstoff weitere Substanzen enthält.

11. Verfahren gemäss Anspruch 10, **dadurch gekennzeichnet, dass** der eingesetzte Wasserstoff eine Reinheit größer als 65 Vol.-% aufweist.

12. Verfahren gemäss den Ansprüchen 1 bis 11, **dadurch gekennzeichnet, dass** das aus der Schwefelwasserstoffsynthese austretende Reaktionsgemisch neben Schwefelwasserstoff Nebenprodukte oder Ausgangsstoffe enthält.

13. Verfahren gemäss den Ansprüchen 1 bis 12, **dadurch gekennzeichnet, dass** man von dem aus der Schwefelwasserstoffsynthese austretenden Reaktionsgemisch vor der Vermischung mit Methanol den darin enthaltenen Schwefel unter Druck abtrennt.

14. Verfahren gemäss Anspruch 13, **dadurch gekennzeichnet, dass** man das Gemisch während oder vor der Abtrennung des Schwefels und gegebenenfalls weiterer schwefelhaltiger Verbindungen auf maximal ~120°C abkühlt.

15. Verfahren gemäss Anspruch 13, **dadurch gekennzeichnet, dass** man das Schwefelwasserstoff enthaltende Reaktionsgemisch nach dem Reaktor bei einem Druck zwischen 9 und 20 bar abkühlt und flüssiger Schwefel abtrennt bzw. rückführt.

16. Verfahren gemäss Anspruch 13, **dadurch gekennzeichnet, dass** zur Reinigung des Schwefelwasserstoffs nach der Schwefelwasserstoffsynthese zwischen beiden Verfahren Adsorbentien bei einem Druck zwischen 9 und 20 bar verwendet werden.

## Claims

1. Process for preparing methyl mercaptan, **characterized in that** the syntheses of hydrogen sulphide and methyl mercaptan are coupled to one another by mixing the reaction mixture which leaves the reactor for hydrogen sulphide synthesis under pressure with methanol and introducing it into the reactor for methyl mercaptan synthesis under pressure, a pressure difference being established between the reactors used for the two syntheses which allows the hydrogen sulphide/methanol mixture to flow in the direction of the methyl mercaptan reactor.

2. Process according to Claim 1, **characterized in that** the pressure difference in the reactors used for the two syntheses is > 0 to < 1 bar, the higher pressure being in the reactor for hydrogen sulphide synthesis.

3. Process according to Claims 1 and 2, **characterized in that** the pressure in the reaction vessels in both processes is more than 8 bar.

4. Process according to Claims 1 to 3, **characterized in that** the pressure in the reactor for hydrogen sulphide synthesis is > 9 to 20 bar and that in the methyl mercaptan reactor is 9 to < 20 bar.

5. Process according to Claims 1 to 4, **characterized in that** the synthesis of hydrogen sulphide is effected at a temperature between 300 and 500°C.

6. Process according to Claims 1 to 5, **characterized in that** the synthesis of hydrogen sulphide is effected in the presence of a heterogeneous catalyst.

7. Process according to Claim 6, **characterized in that** the synthesis of hydrogen sulphide is effected in the presence of a heterogeneous hydrogenation catalyst.

8. Process according to Claim 6, **characterized in that** the synthesis of hydrogen sulphide is effected in the presence of a heterogeneous supported catalyst which comprises one or more of the active elements selected from the group of molybdenum, nickel, tungsten, vanadium, cobalt, sulphur, selenium, phosphorus, arsenic, antimony, bismuth, silicon, aluminium, titanium and zirconium.

9. Process according to Claims 1 to 8, **characterized in that** the hydrogen sulphide is prepared in two or more reactors connected in series.

10. Process according to Claims 1 to 9, **characterized in that** the hydrogen used to prepare hydrogen sulphide comprises further substances.

11. Process according to Claim 10, **characterized in that** the hydrogen used has a purity greater than 65% by volume.

12. Process according to Claims 1 to 11, **characterized in that** the reaction mixture leaving the hydrogen sulphide synthesis comprises by-products or starting materials in addition to hydrogen sulphide.

13. Process according to Claims 1 to 12, **characterized in that** the sulphur present in the reaction mixture leaving the hydrogen sulphide synthesis is removed therefrom under pressure before the mixing with methanol.

14. Process according to Claim 13, **characterized in that** the mixture is cooled to not more than ~ 120°C during or before the removal of the sulphur and any further sulphur compounds.

15. Process according to Claim 13, **characterized in that** the hydrogen sulphide-containing reaction mixture, downstream of the reactor, is cooled at a pressure between 9 and 20 bar and liquid sulphur is removed or recycled.

16. Process according to Claim 13, **characterized in that** the hydrogen sulphide is purified after the hydrogen sulphide synthesis between the two processes by using adsorbents at a pressure between 9 and 20 bar.

## Revendications

1. Procédé de fabrication de méthylmercaptan, **caractérisé en ce que** les synthèses de sulfure d'hydrogène et de méthylmercaptan sont couplées l'une avec l'autre par mélange du mélange réactionnel sous pression sortant du réacteur pour la synthèse de sulfure d'hydrogène avec du méthanol et introduction sous pression dans le réacteur pour la synthèse de méthylmercaptan, une différence de pression qui permet l'écoulement du mélange sulfure d'hydrogène/méthanol dans la direction du réacteur de méthylmercaptan étant ajustée entre les réacteurs utilisés pour les deux synthèses.

2. Procédé selon la revendication 1, **caractérisé en ce que** la différence de pression dans les réacteurs utilisés pour les deux synthèses est > 0 et < 1 bar, la pression la plus élevée régnant dans le réacteur pour la synthèse de sulfure d'hydrogène.

3. Procédé selon la revendication 1 ou 2, **caractérisé en ce que** la pression dans les récipients de réaction des deux procédés est supérieure à 8 bar.

4. Procédé selon les revendications 1 à 3, **caractérisé en ce que** la pression dans le réacteur pour la synthèse de sulfure d'hydrogène est de > 9 à 20 bar et dans le réacteur de méthylmercaptan de 9 à < 20 bar.

5. Procédé selon les revendications 1 à 4, **caractérisé en ce que** la synthèse de sulfure d'hydrogène a lieu à une température comprise entre 300 et 500 °C.

6. Procédé selon les revendications 1 à 5, **caractérisé en ce que** la synthèse de sulfure d'hydrogène a lieu en présence d'un catalyseur hétérogène.

7. Procédé selon la revendication 6, **caractérisé en ce que** la synthèse de sulfure d'hydrogène a lieu en présence d'un catalyseur d'hydrogénation hétérogène.

8. Procédé selon la revendication 6, **caractérisé en ce que** la synthèse de sulfure d'hydrogène a lieu en présence d'un catalyseur supporté hétérogène qui contient un ou plusieurs éléments actifs choisis dans le groupe constitué par le molybdène, le nickel, le tungstène, le vanadium, le cobalt, le soufre, le sélénium, le phosphore, l'arsenic, l'antimoine, le bismuth, le silicium, l'aluminium, le titane et le zirconium.

9. Procédé selon les revendications 1 à 8, **caractérisé en ce que** le sulfure d'hydrogène est fabriqué dans deux réacteurs ou plus connectés en série.

10. Procédé selon les revendications 1 à 9, **caractérisé en ce que** l'hydrogène utilisé pour la fabrication de sulfure d'hydrogène contient des substances supplémentaires.

11. Procédé selon la revendication 10, **caractérisé en ce que** l'hydrogène utilisé présente une pureté de plus de 65 % en volume.

12. Procédé selon les revendications 1 à 11, **caractérisé en ce que** le mélange réactionnel sortant de la synthèse de sulfure d'hydrogène contient des produits secondaires ou des matières premières en plus du sulfure d'hydrogène.

13. Procédé selon les revendications 1 à 12, **caractérisé en ce que** le soufre contenu dans le mélange réactionnel sortant de la synthèse de sulfure d'hydrogène est séparé de celui-ci sous pression avant le mélange avec le méthanol.

14. Procédé selon la revendication 13, **caractérisé en ce que** le mélange est refroidi à au plus ~120 °C pendant ou avant la séparation du soufre et éventuellement d'autres composés contenant du soufre.

15. Procédé selon la revendication 13, **caractérisé en ce que** le mélange réactionnel contenant du sulfure d'hydrogène est refroidi après le réacteur à une pression comprise entre 9 et 20 bar et le soufre liquide est séparé ou recyclé.

16. Procédé selon la revendication 13, **caractérisé en ce que** des adsorbants sont utilisés à une pression comprise entre 9 et 20 bar pour la purification du sulfure d'hydrogène après la synthèse de sulfure d'hydrogène entre les deux procédés.
